# EUROPEAN PATENT SPECIFICATION

(11) **EP 3 174 527 B1**
(45) Date of publication and mention of the grant of the patent: **17.02.2021**
(21) Application number: 15751191.6
(22) Date of filing: 31.07.2015
(51) Int. Cl.: A61P 29/00, A61K 31/192, A61K 9/20, A61K 9/14

(54) **DOSAGE FORM OF SODIUM IBUPROFEN DIHYDRATE ON MESOPOROUS SILICA**
DOSIERUNGSFORM VON NATRIUM IBUPROFEN DIHYDRAT ADSORBIERT AUF MESOPORÖSEM SILIKA
FORME GALÉNIQUE DU DIHYDRATE D'IBUPROFÈNE DE SODIUM ADSORBÉ SUR DU SILICA MESOPOREUX

(30) Priority: 01.08.2014 US 201462032029 P
(43) Date of publication of application: 07.06.2017
(73) Proprietor: Johnson & Johnson Consumer Inc., Skillman, NJ 08558 (US)
(72) Inventor: LEE, Der-Yang, Skillman, New Jersey 08558 (US); KUTCH, Timothy, Skillman, New Jersey 08558 (US)
(74) Representative: Carpmaels & Ransford LLP
(86) International application number: PCT/US2015/043127
(87) International publication number: WO 2016/019252

(56) References cited:
- EP-A1- 2 072 042
- EP-A1- 2 236 130
- EP-A1- 2 324 854
- WO-A2-2007/108010
- WO-A2-2008/104852
- US-A1- 2013 136 793

## Description

### BACKGROUND OF THE INVENTION

### Field of the Invention

The present invention relates to a process for adsorbing sodium ibuprofen dihydrate onto a substrate, a dosage form manufactured by the process and a sodium ibuprofen dosage form for use in a method of reducing a sensation of burning in the mouth or throat of a subject as defined in the appended claims.

Various methods have been used to improve drug delivery of a dosage form. For example, various polymer systems have been tried. The end result has not always been always been favorable and has at times led to poor dissolution or release of the active ingredient.

For example, there is great desire to improve the drug delivery for anti-inflammatory and analgesic drugs, such as steroidal anti-inflammatories, non-steroidal anti-inflammatories (NSAIDs). Often this means fast acting and/or long lasting pain relief. However, achieving these characteristics is difficult.

There is therefore a need for an improved method for loading an active ingredient onto an inert substrate, which does not hinder the release of the active ingredient. The present invention provides such means.

### SUMMARY OF THE INVENTION

The present invention is directed to a process for adsorbing sodium ibuprofen dehydrate onto a substrate, comprising the steps of (a) adding and mixing an inert adsorbent to a non-solid form comprising the sodium ibuprofen dehydrate, thereby forming a mixture; and (b) drying the mixture to form a solid crumbly material; wherein the inert adsorbent is mesoporous silica.

The present invention is also directed to a dosage form manufactured by the process of the invention.

The present invention also includes a sodium ibuprofen dosage form for use in a method of reducing a sensation of burning in the mouth or throat of a subject when swallowing an ibuprofen dosage form; wherein said method comprises the steps of (1) providing the subject with a sodium ibuprofen dosage form and (2) instructing the subject to swallow the sodium ibuprofen dosage form of step (1); wherein the sodium ibuprofen dosage form is made by adsorbing sodium ibuprofen dihydrate onto a substrate, comprising the steps of (a) adding and mixing an inert adsorbent to a non-solid form comprising the sodium ibuprofen dihydrate, thereby forming a mixture; and (b) drying the mixture to form a solid crumbly material; wherein the inert adsorbent is mesoporous silica.

### BRIEF DESCRIPTION OF THE DRAWINGS

FIG. 1 is a graph depicting the dissolution results of Core Formula A versus Advil® Film-Coated Tablet (powered by Advil Ion Core™ Technology) in 0.25% SLS / 0.1N HCl;
FIG. 2 is a graph depicting the dissolution results of Core Formula B and C versus Advil® Film-Coated Tablet (powered by Advil Ion Core™ Technology) in FaSSGF Biorelevant pH 1.6 Media;
FIG. 3 is a graph depicting the average dissolution results of Core Formula B and C versus Advil® Film-Coated Tablet (powered by Advil Ion Core™ Technology) in FaSSGF Biorelevant pH 1.6 Media;

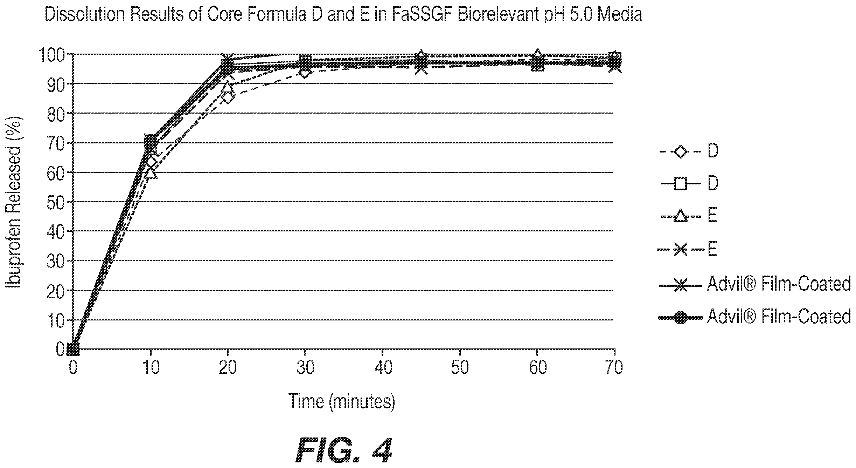

FIG. 4 is a graph depicting the dissolution results of Core Formula D and E versus Advil® Film-Coated Tablet (powered by Advil Ion Core™ Technology) in FeSSGF Biorelevant pH 5.0 Media; and

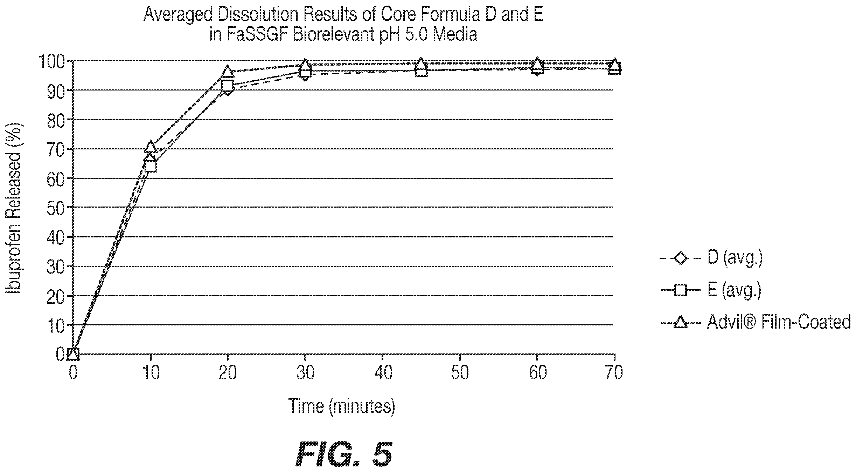

FIG. 5 is a graph depicting the average dissolution results of Core Formula D and E versus Advil® Film-Coated Tablet (powered by Advil Ion Core™ Technology) in FeSSGF Biorelevant pH 5.0 Media.

### DETAILED DESCRIPTION OF THE INVENTION

The present invention is directed to a method for adsorbing sodium ibuprofen dihydrate onto a substrate, comprising the steps of: (a) adding and mixing an inert adsorbent to a non-solid form comprising the sodium ibuprofen dihydrate, thereby forming a mixture; and (b) drying the mixture to form a solid crumbly material; wherein the inert adsorbent is mesoporous silica.

Any suitable means for mixing may be used to mix the inert adsorbent to the non-solid form. For example, the inert adsorbent may be added to the non-solid dry form in a mixing kettle using an impeller mixer.

Additionally, if so desired, the process may include the step of adding additional active pharmaceutical ingredient to the mixture prior to drying the mixture. This optional step would further add to the drug loading.

The resulting mixture may be further subjected to a wet screening step. The wet screening step helps spread out the mixture, which facilitates drying, and may also help reduce the particle size of the mixture of the resulting solid crumbly material.

Drying of the mixture is performed using any suitable means. For example, the mixture may be air dried, oven dried, or fluid bed drying.

In addition, the solid crumbly material may be further processed to reduce the particle size of the material. Any suitable process may be used to reduce the particle size.

For example, the particle size reduction apparatus may be a dry screening apparatus.

In addition, a glidant such as colloidal silicon dioxide, may be added before, after or during the particle size reduction step.

Afterward, the resulting material may be compressed to form a core.

The non-solid form may be, for example, a solution, a suspension, an emulsion, a paste, or slurry. Additionally, the non-solid form may be aqueous based, solvent based or lipid based.

In one embodiment, the adsorption is performed at temperatures ranging from about 50°C to about 80°C, preferably, from about 50°C to about 60°C. The range of drug potency was 40-95%.

The active pharmaceutical ingredient ("API") is sodium ibuprofen dihydrate

The resulting material may be compressed to form cores. The amount of API in the core will depend on the API itself and the desired dosage level. Preferably, the core has about 1 mg to about 500 mg, and more preferably, about 1 mg to about 400 mg of the API. Even more preferably, the core has 50 mg to about 400 mg, and still even more preferably, about 150 mg to about 400 mg of the API.

The inert adsorbent is mesoporous silica, which has a higher bulk density and uniform particle size distribution as compared to fumed silica with a unique morphology. Mesoporous silica may be sourced from W.R. Grace and Company of Maryland, which markets its product under the tradename SYLOID®.

The core includes from about 5 to about 95 weight percent (wt.%) of the inert adsorbent.

In one embodiment, the core includes from about 5 to about 90 wt.% of the inert adsorbent. Preferably, the core includes from about 5 to about 75 wt.% of the inert adsorbent. More preferably, the core includes from about 5 to about 50 wt.% of the inert adsorbent. Even more preferably, the core includes from about 5 to about 20 wt.% of the inert adsorbent.

In an alternative embodiment, the core includes from about 50 to about 95 wt.% of the inert adsorbent. Preferably, the core includes from about 50 to about 90 wt.% of the inert adsorbent. More preferably, the core includes from about 75 to about 90 wt.% of the inert adsorbent.

In one embodiment, the dried solid crumbly material of the present invention may be used in a core. The core may be compressed in the form of a tablet or as at least one layer of a multilayer tablet. In another embodiment the dried solid crumbly material may be deposited into a capsule form. In another embodiment, the dried solid crumbly material may be used for direct administration from a sachet.

Optionally, other ingredients may be included in the composition or dosage form of the present invention.

For example, a glidant may also be included in the core composition to assist in the flow properties of the composition. Suitable glidants include, for example, silicon dioxide such as colloidal silica, fumed silica, mixtures thereof, and the like.

In one embodiment, the core may include from about 0.01 to about 3 wt.% of the glidant. In another embodiment, the core includes from about 0.05 to about 2 wt.% of the glidant. In yet another embodiment, the core includes from about 0.1 to about 1 wt.% of the glidant.

Other ingredients or components that may be added to the composition include, but are not limited to, superdisintegrants, lubricants, aromas; sweeteners such as, sorbitol, sugar, and high intensity sweeteners such as sucralose, aspartame and saccharine and the like may be included.

Any coloring agent suitable for use in a food or pharmaceutical product may be used in the present inventive composition. Typical coloring agents include, for example, azo dyes, quinopthalone dyes, triphenylmethane dyes, xanthene dyes, indigoid dyes, iron oxides, iron hydroxides, titanium dioxide, natural dyes, and mixtures thereof. More specifically, suitable colorants include, but are not limited to patent blue V, acid brilliant green BS, red 2G, azorubine, ponceau 4R, amaranth, D&C red 33, D&C red 22, D&C red 26, D&C red 28, D&C yellow 10, FD&C yellow 5, FD&C yellow 6, FD&C red 3, FD&C red 40, FD&C blue 1, FD&C blue 2, FD&C green 3, brilliant black BN, carbon black, iron oxide black, iron oxide red, iron oxide yellow, titanium dioxide, riboflavin, carotenes, antyhocyanines, turmeric, cochineal extract, clorophyllin, canthaxanthin, caramel, betanin, and mixtures thereof.

Similarly, a flavor may be included in the composition or solid dosage form. The amount of flavor added to the composition will be dependent upon the desired taste characteristics.

The present disclosure includes a method of taste masking a sodium ibuprofen dosage form. The method comprising the steps of adsorbing sodium ibuprofen dihydrate onto a substrate, comprising the steps of (a) adding and mixing an inert adsorbent to a non-solid form comprising the sodium ibuprofen dihydrate, thereby forming a mixture; and (b) drying the mixture to form a solid crumbly material.

The present disclosure further includes a method of reducing a sensation of burning in the mouth or throat of a subject when swallowing an ibuprofen dosage form. The method comprising the steps of (1) providing the subject with a sodium ibuprofen dosage form, wherein the sodium ibuprofen dosage form is made by adsorbing sodium ibuprofen dihydrate onto a substrate, comprising the steps of (a) adding and mixing an inert adsorbent to a non-solid form comprising the sodium ibuprofen dihydrate, thereby forming a mixture; and (b) drying the mixture to form a solid crumbly material; and (2) instructing the subject to swallow the sodium ibuprofen dosage form of step (1).

The following examples are provided to further illustrate the compositions and methods of the present invention.

### EXAMPLE 1

TABLE 1

**Solubility of Sodium Ibuprofen Dihydrate in Water:**

| **Temperature** | R.T. (∼25°C) | 40°C | 50°C* | 60°C |
|---|---|---|---|---|
| **Solubility (mg/mL)** | 250 | 551 | 653 | 676 |

| | | | | |
|---|---|---|---|---|
| *This temperature was selected for use as part of the adsorption procedure (reference: Step #1) | | | | |

**Suspension/Slurry Procedure:**
1. 23 mL of sterile water was heated to a temperature of 50°C using a water bath.
2. 45 grams of sodium ibuprofen dihydrate was added to the heated water and dissolved for approximately 4 minutes. The material was viscous and white in color due to being above the saturation point.
TABLE 2

**Core Formula A:**

| **Material** | **%w/w** |
|---|---|
| Sodium ibuprofen dihydrate | 89.55 |
| Mesoporous silicon dioxide (Syloid® XDP 3150) | 9.95 |
| Silicon dioxide (Cab-O-Sil® M-5) | 0.50 |
| **TOTAL** | **100.00** |

**Adsorption Procedure:**
1. Using an impeller, the suspension/slurry was mixed at the slowest mixing speed possible while 5 grams of mesoporous silicon dioxide (Syloid® XDP 3150) was added. The resulting mixture was more viscous and was able to form soft peaks. Total mixing time was not more than 30 seconds.
2. The material was wet-sieved through a #30 mesh sieve. The material hung from the underside of the sieve and appeared as small tubes with an extruded appearance.
3. The extrudates were allowed to air dry for approximately 6 minutes, then scraped off the sieve and dried overnight for about 20 hours, resulting in a dry solid crumbly material.
4. The cylinders were dry-sieved through a #60 mesh sieve and blended with silicon dioxide.
TABLE 3

**Assay Results:**

| **Adsorbent** | **Drug Potency (%w/w)** | **Drug Recovery (%w/w)** |
|---|---|---|
| Neusilin® US2 | 73 | 101.1 |
| Syloid® XDP 3050 | 63 | 103.3 |
| Syloid® XDP 3150 | 63 | 98.3 |
| Syloid® XDP 3050 | 90 | 100.2 |
| Syloid® XDP 3150 | 90 | 99.6 |

| | | |
|---|---|---|
| - Neusilin® US2 - magnesium aluminometasilicate from Fuji Chemical Industry Co. Ltd. - Syloid® XDP 3050 and Syloid® XDP 3150 are mesoporous silicas from W.R. Grace | | |

The process of the present invention demonstrates the ability to formulate sodium ibuprofen dihydrate particles with good flow characteristics.
TABLE 4

**Formulae for Drug Loaded Particles B, C, D, and E:**

| **Material** | **B (%w/w)** | | **C (%w/w)** | | **D (%w/w)** | | **E (%w/w)** | |
|---|---|---|---|---|---|---|---|---|
| | **g** | **% w/w** | **g** | **% w/w** | **g** | **% w/w** | **g** | **% w/w** |
| Sodium ibuprofen dihydrate | | | | | | | | |
| Solution | 74.75 | 29.9 | 74.75 | 29.9 | 74.75 | 29.9 | 74.75 | 29.9 |
| Powder | 139.00 | 55.6 | 127.75 | 51.1 | 139.00 | 55.6 | 127.75 | 51.1 |
| Syloid® XDP 3150 | 23.75 | 9.5 | 22.50 | 9.0 | 23.75 | 9.5 | 22.50 | 9.0 |
| Avicel® PH102 | 12.50 | 5.0 | 25.00 | 10.0 | - | - | - | - |
| ProSolv® SMCC 90 | - | - | - | - | 12.50 | 5.0 | 25.00 | 10.0 |
| Water (removed during processing) | 115.00 | N/A | 115.00 | N/A | 115.00 | N/A | 115.00 | N/A |
| TOTAL | 250.00 | 100 | 250.00 | 100 | 250.00 | 100 | 250.00 | 100 |

TABLE 5

**Particle Size Distribution for Drug Loaded Particles B, C, D, and E:**

| **Mesh** | **Size (um)** | **B** | | **C** | | **D** | **E** |
|---|---|---|---|---|---|---|---|
| | | **#30 wet/ #30 dry** | **#30 wet/ #40 dry** | **#30 wet/ #30 dry** | **#30 wet/ #40 dry** | **#30 wet/ #60 dry** | **#30 wet/ #60 dry** |
| 45 | 354 | 32.6 | 4.2 | 24.9 | 3.3 | 0.2 | 0.3 |
| 60 | 250 | 7.7 | 10.5 | 7.0 | 10.0 | 0.3 | 0.6 |
| 100 | 150 | 14.5 | 20.8 | 16.9 | 20.9 | 30.9 | 30.8 |
| 170 | 90 | 26.8 | 37.7 | 28.7 | 36.9 | 42.1 | 40.4 |
| 200 | 75 | 6.8 | 9.3 | 7.6 | 8.9 | 9.9 | 9.5 |
| 325 | 45 | 9.0 | 13.4 | 10.8 | 14.5 | 13.6 | 1 2.8 |
| Pan | 0 | 2.4 | 4.1 | 4.1 | 5.4 | 3.0 | 5.6 |
| Bulk (g/mL) | N/A | 0.2857 | 0.2545 | 0.2859 | 0.2932 | 0.2767 | 0.2781 |
| Tap (g/mL) | N/A | 0.4255 | 0.4073 | 0.4229 | 0.4280 | 0.4125 | 0.4040 |
| LOD, n=3 (StDev) | N/A | - | - | - | - | 12.659% (0.193%) | 12.135% (0.027%) |

TABLE 6

**Core Formula for B, C, D, and E:**

| **Material** | **B (%w/w)** | **C (%w/w)** | **D (%w/w)** | **E (%w/w)** |
|---|---|---|---|---|
| Sodium ibuprofen dihydrate | | | | |
| Solution | 29.15 | 29.15 | 29.15 | 29.15 |
| Powder | 54.21 | 49.82 | 54.21 | 49.82 |
| Syloid® XDP 3150 | 9.26 | 8.78 | 9.26 | 8.78 |
| Avicel® PH102 | 4.88 | 9.75 | -- | -- |
| ProSolv® SMCC 90 | -- | -- | 4.88 | 9.75 |
| Syloid® 63FP | 2.00 | 2.00 | 2.00 | 2.00 |
| Magnesium stearate | 0.50 | 0.50 | 0.50 | 0.50 |
| **TOTAL** | **100.00** | **100.00** | **100.00** | **100.00** |

| | | | | |
|---|---|---|---|---|
| - Avicel® PH102 - microcrystalline cellulose supplied by FMC BioPolymer of Philadelphia, PA - ProSolv® SMCC 90 - blend of silicified microcrystalline cellulose (98%) and colloidal silicon dioxide (2%) supplied by JRS Pharma of Rosenberg, Germany - Syloid® 63FP is a silica from W.R. Grace | | | | |

**Adsorption Procedure for Formula B, C, D, and E:**
1. Sterile water was heated to a temperature of 50°C using a water bath.
2. Syloid® XDP 3150 and ProSolv® SMCC90 or Avicel® PH102 were premixed in a polyethylene bag.
3. "Solution" portion (29.15g) of sodium ibuprofen dihydrate was added to the heated water (44.6 ml) and dissolved for approximately 4 minutes. The resulting drug solution was clear and watery.
4. The drug solution was removed from the water bath and the premixed dry materials from Step #2 were added to the drug solution from Step #3. A metal spatula was used to incorporate and ensure all materials were wetted.
5. The intermediate product batch of Step #4, was covered tightly with aluminum foil and soaked on lab bench at room temperature for N.M.T. 30 minutes.
6. The intermediate product batch was returned to a water bath and mixed using a metal spatula to ensure any unabsorbed drug was in solution.
7. An impeller mixer was used to mix the slurry from Step # 6 while adding the "powder" portion (see Table 6 for level of powder) of sodium ibuprofen dihydrate for N.M.T. 40 seconds. The material was more viscous than in Step #6 and was able to form soft peaks. Total mixing time was N.M.T. 30 seconds.
8. The material was wet-sieved through a #30 mesh sieve. Material hung from the underside of the sieve and appeared as small tubes with an extruded appearance.
9. The extrudates were allowed to air dry for approximately 6 minutes, then scrapped off the sieve and spread on tray paper, where it was placed in a laboratory fume hood to dry at ambient temperature for about 20 hours, resulting in a dry solid crumbly material.
10. The cylinder were dry-sieved through a #60 mesh sieve and blended with Syloid®63FP and magnesium stearate.
11. The blend from Step 10 was compressed into tablets using the procedure below.

**Compression of Tablets:**
A:
   Punch- 5/16" round flat-face bevel-edge
   Force- 0.75 tons
   Dose- 256.24mg sodium ibuprofen dihydrate (equivalent to 200mg ibuprofen acid)
B, C, D, and E:
   Punch- 5/16" round flat-face bevel-edge
   Force- 1.0 tons
   Dose- 128.12mg sodium ibuprofen dihydrate (equivalent to 100mg ibuprofen acid)

### EXAMPLE 2

Dissolution results compared to Advil® Film-Coated powered by Advil Ion Core™ Technology in 0.25% SLS (sodium lauryl sulfate) / 0.1N HCl media.

Using HPLC method at 220nm and 50rpm paddles. The injection volume was 10 µL for samples ranging from 0.1 mg/mL to 0.3 mg/mL (100mg Sodium IBU tablet in 1000mL dissolution vessel is 0.1 mg/mL that was injected without any further dilutions).
TABLE 7

**Dissolution Results of Core Formula A in 0.25% SLS / 0.1N HCl**

| | **Time (Minutes)** | | | | | |
|---|---|---|---|---|---|---|
| **Sample** | **10** | **20** | **30** | **45** | **60** | **90** |
| **A** | 70.550 | 76.990 | 80.562 | 82.735 | 84.986 | 97.620 |
| **A** | 53.725 | 80.533 | 74.871 | 80.015 | 82.153 | 94.780 |
| **A** | 71.740 | 82.814 | 86.2.80 | 90.376 | 89.694 | 95.771 |
| Advil® Film-Coated | 22.818 | 44.956 | 70.404 | 80.452 | 83.561 | 95.778 |
| Advil® Film-Coated | 5.277 | 36.931 | 69.429 | 81.811 | 86.421 | 99.252 |
| Advil® Film-Coated | 18.331 | 53.485 | 72.402 | 81.455 | 84.463 | 95.830 |

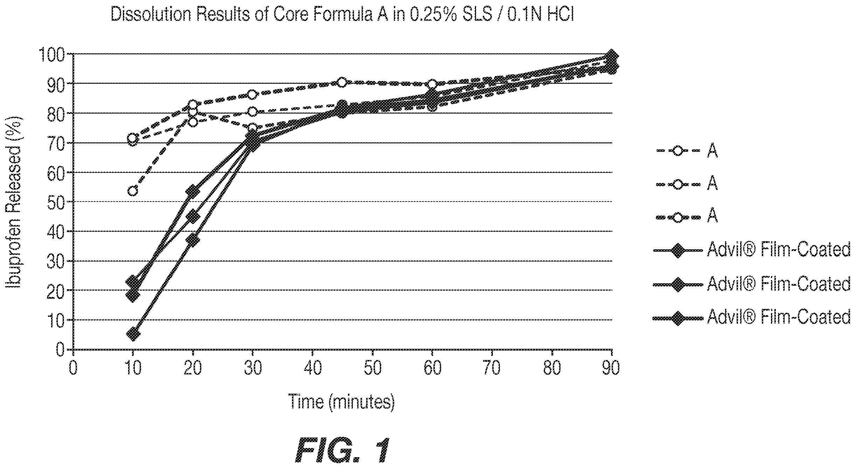

FIG. 1 provides a graphical depiction of the results, which show at 10 minutes, the Formulation A samples were at least 50% released, whereas the Advil® Film-Coated powered by Advil Ion Core™ Technology product was less than 23% released. At 20 minutes, the Formulation A samples were at least 75% released, whereas the Advil® Film-Coated powered by Advil Ion Core™ Technology product was less than 54% released.

Dissolution results compared to Advil® Film-Coated Tablet (powered by Advil Ion Core™ Technology) in FaSSGF (Fast State Simulated Gastric Fluid) Biorelevant pH 1.6 media.

Using HPLC method at 220nm and 75rpm paddles. The injection volume was 10 µL for samples ranging from 0.1 mg/mL to 0.3 mg/mL (100mg Sodium IBU tablet in 1000mL dissolution vessel is 0.1 mg/mL that was injected without any further dilutions).
TABLE 8

**Dissolution Results of Core Formula B and C in FaSSGF Biorelevant pH 1.6 media**

| | **Time (minutes)** | | | | | |
|---|---|---|---|---|---|---|
| **Sample** | **10** | **20** | **30** | **45** | **60** | **70** |
| B | 25.0 | 40.5 | 49.6 | 57.6 | 61.3 | 63.1 |
| B | 25. 0 | 45.7 | 54.8 | 61.4 | 65.1 | 67.2 |
| C | 22.5 | 39.6 | 50.2 | 57.6 | 61.5 | 62.4 |
| C | 23.7 | 40.1 | 51.3 | 58.6 | 61.8 | 62.5 |
| Advil® Film-Coated | 12.8 | 32.4 | 44.6 | 56.2 | 59.6 | 63.0 |
| Advil® Film-Coated | 8.0 | 34.1 | 53.0 | 58.5 | 60.8 | 60.8 |

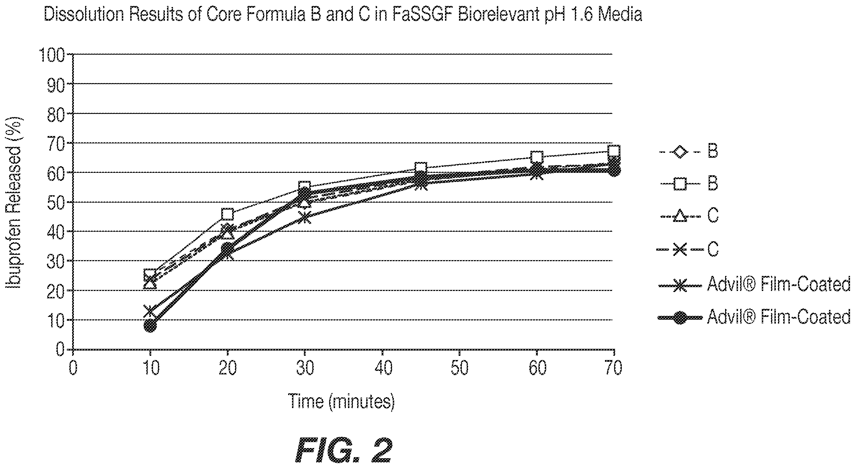

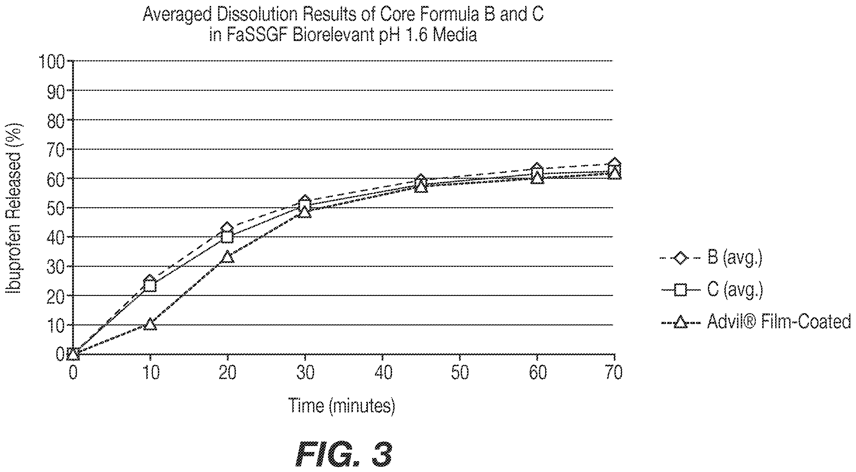

FIG. 2 and FIG. 3 provide graphical depictions of the results, which show at 10 minutes, the Formulation B and C samples were at least 22% released, whereas the Advil® Film-Coated Tablet (powered by Advil Ion Core™ Technology) product was less than 13% released. At 20 minutes, the Formulation B and C samples were at least 35% released, whereas the Advil® Film-Coated Tablet (powered by Advil Ion Core™ Technology) product was less than 35% released.

Dissolution results compared to Advil® Film-Coated Tablet (powered by Advil Ion Core™ Technology) in FeSSGF (Fed State Simulated Gastric Fluid) Biorelevant pH 5.0 media.

Using HPLC method at 220nm and 75rpm paddles. The injection volume was 10 µL for samples ranging from 0.1 mg/mL to 0.3 mg/mL (100mg Sodium IBU tablet in 1000mL dissolution vessel is 0.1 mg/mL that was injected without any further dilutions).
TABLE 9

**Dissolution Results of Core Formula D and E in FeSSGF Biorelevant pH 5.0 media**

| | **Time (minutes)** | | | | | |
|---|---|---|---|---|---|---|
| **Sample** | **10** | **20** | **30** | **45** | **60** | **70** |
| D | 63.6 | 85.3 | 93.8 | 97.1 | 97.7 | 97.2 |
| D | 68.2 | 95.6 | 96.9 | 97.6 | 96.5 | 98.0 |
| E | 59.9 | 88.9 | 98.0 | 99.0 | 99.1 | 98.8 |
| E | 68.0 | 93.4 | 95.6 | 95.5 | 97.0 | 95.9 |
| Advil® Film-Coated | 70.8 | 97.9 | 100.8 | 100.9 | 101.2 | 101.0 |
| Advil® Film-Coated | 70.6 | 95.0 | 96.3 | 97.0 | 97.0 | 96.9 |

### Preparation of Biorelevant buffers

**FaSSGF Biorelevant pH 1.6 media-** Dissolved 0.32g lecithin in 3.2 mL of dichloromethane and 0.42g of sodium taurocholate in 10L of water. Add 2g pepsin and 40g of NaCl. Heat mixture to 40°C and q.s. to 20L with water.

**TABLE 10**

| **Composition** | |
|---|---|
| Sodium taurocholate | 80µM |
| Lecithin | 20 µM |
| Pepsin | 0.1 mg/mL |
| Sodium chloride | 34.2 mM |
| Hydrochloric acid q.s. | pH = 1.6 |

| | |
|---|---|
| Ref: Karuppiah, V.; Kannappan, N.; Manavalan, R. In-vitro and Simulated In-vivo Dissolution of Dipyridamole Extended Release Capsules. Intl. J. Pharm. Sciences Review and Research. 13(1), 68-72. (2012) | |

**FeSSGF Biorelevant pH 5.0 media -** Dissolved 277.0g NaCl and 80.08g sodium acetate in 10L of water. Added 20mL of acetic acid and q.s. to 20L with water.

**TABLE 11**

| **Composition** | |
|---|---|
| Sodium chloride | 237.02 mM |
| Acetic acid | 17.12 mM |
| Sodium acetate | 29.75 mM |
| Acetate buffer | 20 mL |
| Hydrochloric acid q.s. | pH = 5.0 |

| | |
|---|---|
| Ref: Karuppiah, V.; Kannappan, N.; Manavalan, R. In-vitro and Simulated In-vivo Dissolution of Dipyridamole Extended Release Capsules. Intl. J. Pharm. Sciences Review and Research. 13(1), 68-72. (2012) | |

### EXAMPLE 3

### TASTE-TESTING

Sodium ibuprofen active pharmaceutical ingredient (API) loaded particles were prepared for taste-testing using the formula in Table 12. The adsorption procedure was the same as described in Example 1.
TABLE 12

**Particle Formulation For Taste-Testing**

| **Material** | **Amount (g)** | **%w/w** |
|---|---|---|
| Sodium ibuprofen dihydrate | 8 | 40 |
| Syloid® XDP 3150 | 12 | 60 |
| Water* | 32 | -- |
| **TOTAL** | **20** | **100%** |

| | | |
|---|---|---|
| *theoretically completely removed during drying step in the fume hood | | |

The particles containing sodium ibuprofen were then tested for taste sensation. The particles were compared to pure sodium ibuprofen API (active pharmaceutical ingredient). Each sample contained 50 mg of sodium ibuprofen. Four subjects were instructed to ingest each sample and evaluate the sensation based on several criteria; including bitterness, saltiness and mouth/throat burn. The scale was based on a level of 0-10, wherein 0 = None (no bitterness, saltiness or throat/mouth burn) and 10 = High (high level of perceived bitterness, saltiness or throat/mouth burn). The results are presented in Table 13.
TABLE 13

**Results of Taste-Testing**

| | Bitterness | | Saltiness | | Mouth/Throat Burn | |
|---|---|---|---|---|---|---|
| Subject | 40% API Loaded Particles | Pure API | 40% API Loaded Particles | Pure API | 40% API Pure API Loaded Particles | |
| 1 | 6 | 7 | 4 | 5 | 6 | 8 |
| 2 | 1 | 1 | 3 | 5 | 9 | 10 |
| 3 | 0 | 4 | 2 | 1 | 3 | 5 |
| 4 | 3 | 3 | 2 | 5 | 1 | 9 |
| Average | 2.5 | 3.75 | 2.75 | 4 | 4.75 | 8 |

The average sensation change for Bitterness was 1.25; for Saltiness was 1.25; and for Mouth/Throat Burn was 3.25.

In all criteria, the pure API displayed a higher level than the 40% API loaded particles, indicating that the particles provided taste-masking of the sensation.

## Claims

1. A process for adsorbing sodium ibuprofen dihydrate onto a substrate, comprising the steps of:
(a) adding and mixing an inert adsorbent to a non-solid form comprising the sodium ibuprofen dihydrate, thereby forming a mixture; and
(b) drying the mixture to form a solid crumbly material; wherein the inert adsorbent is mesoporous silica.

2. The process of claim 1, wherein the non-solid form is a solution, a suspension, an emulsion, a paste, or slurry.

3. The process of claim 1, further comprising a wet screening step after step (a).

4. The process of claim 1, wherein the solid crumbly material is further processed in a particle size reduction apparatus.

5. The process of claim 1, wherein the suspension is at a temperature of about 50° C to about 60° C prior to the addition of the inert adsorbent.

6. The process of claim 1, further comprising the step of adding additional sodium ibuprofen dihydrate to the mixture, prior to step (b).

7. A dosage form manufactured by the process of claim 1.

8. The dosage form of claim 7, wherein about 50% of the sodium ibuprofen dihydrate is released in about 10 minutes in 0.25% SLS / 0.1N HCl media using an HPLC method.

9. The dosage form of claim 7, wherein about 22% of the sodium ibuprofen dihydrate is released in about 10 minutes in FaSSGF Biorelevant pH 1.6 media using an HPLC method.

10. The dosage form of claim 7, wherein about 35% of the sodium ibuprofen dihydrate is released in about 20 minutes in FaSSGF Biorelevant pH 1.6 media using an HPLC method.

11. A sodium ibuprofen dosage form for use in a method of reducing a sensation of burning in the mouth or throat of a subject when swallowing an ibuprofen dosage form;
wherein said method comprises the steps of (1) providing the subject with a sodium ibuprofen dosage form and (2) instructing the subject to swallow the sodium ibuprofen dosage form of step (1);
wherein the sodium ibuprofen dosage form is made by adsorbing sodium ibuprofen dihydrate onto a substrate, comprising the steps of (a) adding and mixing an inert adsorbent to a non-solid form comprising the sodium ibuprofen dihydrate, thereby forming a mixture; and (b) drying the mixture to form a solid crumbly material;
wherein the inert adsorbent is mesoporous silica.

## Patentansprüche

1. Verfahren zum Adsorbieren von Ibuprofen-Natrium-Dihydrat auf ein Substrat, umfassend die Schritte:
(a) Zugeben und Vermischen eines inerten Adsorptionsmittels zu/mit einer nicht-festen Form, die das Ibuprofen-Natrium-Dihydrat umfasst, um ein Gemisch zu bilden; und
(b) Trocknen des Gemischs, um ein festes, brüchiges Material zu bilden;
wobei das inerte Adsorptionsmittel mesoporöses Siliciumdioxid ist.

2. Verfahren gemäß Anspruch 1, wobei die nicht-feste Form eine Lösung, eine Suspension, eine Emulsion, eine Paste oder eine Aufschlämmung ist.

3. Verfahren gemäß Anspruch 1, ferner umfassend einen Nasssiebungsschritt nach Schritt (a).

4. Verfahren gemäß Anspruch 1, wobei das feste, brüchige Material in einer Partikelgrößenverringerungsvorrichtung weiterverarbeitet wird.

5. Verfahren gemäß Anspruch 1, wobei die Suspension vor dem Zugeben des inerten Adsorptionsmittels bei einer Temperatur von etwa 50 °C bis etwa 60 °C vorliegt.

6. Verfahren gemäß Anspruch 1, ferner umfassend den Schritt des Zugebens von zusätzlichem Ibuprofen-Natrium-Dihydrat zu dem Gemisch vor Schritt (b).

7. Darreichungsform, hergestellt durch das Verfahren gemäß Anspruch 1.

8. Darreichungsform gemäß Anspruch 7, wobei in 0,25%-SLS/0,1N-HCl-Medium unter Verwendung eines HPLC-Verfahrens etwa 50 % des Ibuprofen-Natrium-Dihydrats innerhalb von etwa 10 Minuten freigesetzt werden.

9. Darreichungsform gemäß Anspruch 7, wobei in FaSSGF-Biorelevant-Medium mit pH 1,6 unter Verwendung eines HPLC-Verfahrens etwa 22 % des Ibuprofen-Natrium-Dihydrats innerhalb von etwa 10 Minuten freigesetzt werden.

10. Darreichungsform gemäß Anspruch 7, wobei in FaSSGF-Biorelevant-Medium mit pH 1,6 unter Verwendung eines HPLC-Verfahrens etwa 35 % des Ibuprofen-Natrium-Dihydrats innerhalb von etwa 20 Minuten freigesetzt werden.

11. Ibuprofen-Natrium-Darreichungsform zur Verwendung bei einem Verfahren zum Verringern der Wahrnehmung von Brennen im Mund oder Rachen eines Subjekts beim Schlucken einer Ibuprofen-Darreichungsform;
wobei das Verfahren die Schritte (1) Versorgen des Subjekts mit einer Ibuprofen-Natrium-Darreichungsform und (2) Anweisen des Subjekts, die Ibuprofen-Natrium-Darreichungsform von Schritt (1) zu schlucken, umfasst; wobei die Ibuprofen-Natrium-Darreichungsform durch Adsorption von Ibuprofen-Natrium-Dihydrat auf ein Substrat, umfassend die Schritte (a) Zugeben und Vermischen eines inerten Adsorptionsmittels zu/mit einer nicht-festen Form, die das Ibuprofen-Natrium-Dihydrat umfasst, um ein Gemisch zu bilden; und (b) Trocknen des Gemischs, um ein festes, brüchiges Material zu bilden, hergestellt ist;
wobei das inerte Adsorptionsmittel mesoporöses Siliciumdioxid ist.

## Revendications

1. Procédé pour l'adsorption de dihydrate d'ibuprofène sodique sur un substrat, comprenant les étapes de :
(a) ajout et mélange d'un adsorbant inerte à une forme non solide comprenant le dihydrate d'ibuprofène sodique, formant ainsi un mélange ; et
(b) séchage du mélange pour former un matériau friable solide ;
l'adsorbant inerte étant une silice mésoporeuse.

2. Procédé selon la revendication 1, la forme non solide étant une solution, une suspension, une émulsion, une pâte, ou une bouillie.

3. Procédé selon la revendication 1, comprenant en outre une étape de criblage humide après l'étape (a).

4. Procédé selon la revendication 1, le matériau friable solide étant ensuite traité dans un appareil de réduction de taille de particule.

5. Procédé selon la revendication 1, la suspension étant à une température d'environ 50 °C à environ 60 °C avant l'addition de l'adsorbant inerte.

6. Procédé selon la revendication 1, comprenant en outre l'étape d'ajout de dihydrate d'ibuprofène sodique supplémentaire au mélange, avant l'étape (b).

7. Forme de dosage fabriquée par le procédé selon la revendication 1.

8. Forme de dosage selon la revendication 7, environ 50 % du dihydrate d'ibuprofène sodique étant libérés en environ 10 minutes dans un milieu de 0,25 % SLS/0,1 N HCl à l'aide d'un procédé d'HPLC.

9. Forme de dosage selon la revendication 7, environ 22 % du dihydrate d'ibuprofène sodique étant libérés en environ 10 minutes dans un milieu de type FaSSGF Biorelevant à pH 1,6 à l'aide d'un procédé d'HPLC.

10. Forme de dosage selon la revendication 7, environ 35 % du dihydrate d'ibuprofène sodique étant libérés en environ 20 minutes dans un milieu de type FaSSGF Biorelevant à pH 1,6 à l'aide d'un procédé d'HPLC.

11. Forme de dosage d'ibuprofène sodique pour une utilisation dans un procédé de réduction d'une sensation de brûlure dans la bouche ou la gorge d'un sujet lorsqu'il avale une forme de dosage d'ibuprofène ;
ledit procédé comprenant les étapes de (1) mise à disposition du sujet d'une forme de dosage d'ibuprofène sodique et (2) instruction au sujet d'avaler la forme de dosage d'ibuprofène sodique de l'étape (1) ;
la forme de dosage d'ibuprofène sodique étant faite en adsorbant du dihydrate d'ibuprofène sodique sur un substrat, comprenant les étapes (a) d'ajout et de mélange d'un adsorbant inerte à une forme non solide comprenant le dihydrate d'ibuprofène sodique, formant ainsi un mélange ; et (b) séchage du mélange pour former un matériau friable solide ;
l'adsorbant inerte étant une silice mésoporeuse.
